# EUROPEAN PATENT APPLICATION

(11) **EP 2 116 536 A1**
(43) Date of publication of application: **11.11.2009**
(21) Application number: 08155802.5
(22) Date of filing: 07.05.2008
(51) Int. Cl.: C07D 215/227, A61K 31/4704, A61P 11/00

(54) **Crystal form of 8-hydroxy-5-[(1r)-1-hydroxy-2-[[(1r)-2-(4-methoxyphenyl)-1-methylethyl]amino]ethyl]-2(1h)- quinolinone monohydrochloride**

(71) Applicant: CHIESI FARMACEUTICI S.p.A., I-43100 Parma (IT)
(72) Inventor: Pivetti, Fausto, 43100, PARMA (IT); Lutero, Emilio, 43100, PARMA (IT)
(74) Representative: Minoja, Fabrizio

(57) **Abstract**

The present invention relates to a novel crystal form of 8-hydroxy-5-[(1R)-1-hydroxy-2-[[(1R)-2-(4-methoxyphenyl)-1-methylethyl]amino]ethyl]-2(1H)-quinolinone monohydrochloride.

The invention also relates to processes for its preparation, pharmaceutical compositions thereof, and to its use as a medicament.

## Description

### FIELD OF INVENTION

The present invention relates to a novel crystal form of 8-hydroxy-5-[(1R)-1-hydroxy-2-[[(1R)-2-(4-methoxyphenyl)-1-methylethyl]amino]ethyl]-2(1 H)-quinolinone monohydrochloride.

The invention also relates to processes for its preparation, pharmaceutical compositions thereof, and to its use as a medicament.

### BACKGROUND OF THE INVENTION

8-Hydroxy-5-[(1R)-1-hydroxy-2-[[(1R)-2-(4-methoxyphenyl)-1-methylethyl]amino]ethyl]2(1H)-quinolinone monohydrochloride (I) has been described in EP 147719 as a bronchodilator having a potent beta-2-adrenoceptor stimulating action.

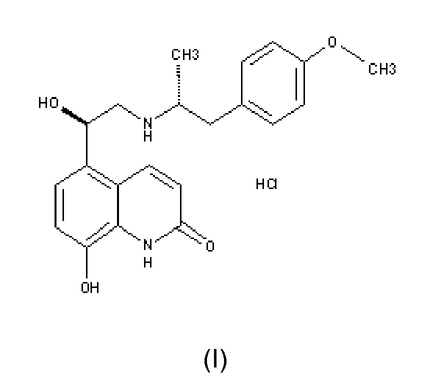

The compound, which has also been referred to the literature with the codes TA 2005 and CHF 4226, may be prepared in a particular prevalently amorphous form in accordance with the method given in Example 4.

This compound, hereinafter referred to as CHF 4226, has been investigated for use as a medicament for treating inflammatory or obstructive airways diseases.

However, for preparing suitable pharmaceutical compositions, it is important that the compound is in a thermodynamically stable crystal form. It is also very important that the compound has good handling qualities and can be easily obtained on a commercial scale.

A thermodynamically stable crystal form of CHF 4226, hereinafter referred to as form A, has been disclosed and closely characterised in WO 2005/089760, but said form is obtained in an adequate pharmaceutical level of chemical purity and crystallinity only by applying specific conditions of crystallization.

Therefore, it would be advantageous to provide further thermodynamically stable crystal forms of CHF 4226, which are easy to obtain by crystallization, and characterised by a high level of chemical purity and crystallinity as well as good handling qualities for pharmaceutical use.

### SUMMARY OF THE INVENTION

The invention relates to a novel crystal form of 8-hydroxy-5-[(1R)-1-hydroxy-2-[[(1R)-2-(4-methoxyphenyl)-1-methylethyl]amino]ethyl]-2(1H)-quinolinone monohydrochloride (CHF 4226), designated crystal form E.

Said form is a thermodynamically stable hemihydrate, i.e. a pseudopolymorph, and is characterised by a high level of chemical purity and crystallinity as well as good handling characteristics for the preparation of pharmaceutical compositions.

The crystal form of the invention may be selectively produced by crystallization from appropriate solvents and conditions and is distinguishable based upon its characteristic peaks in the X-ray powder diffraction (XRPD) pattern, and its characteristic melting range.

Accordingly, the invention is also directed to processes for the preparation of said form comprising crystallization or re-crystallization from appropriate solvents.

The invention is further directed to pharmaceutical compositions comprising CHF 4226 crystal form E, and to its use as a medicament.

The crystal form the invention is preferably administered by inhalation for the prevention and/or treatment of an inflammatory or obstructive airways disease such as asthma or chronic obstructive pulmonary disease (COPD).

Accordingly, in a further aspect, the present invention comprises the use of CHF 4226 crystal form E for the prevention and/or treatment of an inflammatory or obstructive airways disease such as asthma or chronic obstructive pulmonary disease (COPD).

In a still further aspect, the present invention comprises a method of preventing and/or treating an inflammatory or obstructive airways disease such as asthma or chronic obstructive pulmonary disease (COPD), which comprises the inhalatony administration of an effective amount of CHF 4226 crystal form E.

### DEFINITIONS

Unless defined otherwise, all technical and scientific terms used herein have the same meaning as is commonly understood by one of skill in the art to which this subject matter belongs.

As used herein, the term "prevalently amorphous", describes a non-ordered solid state characterised by a diffused X-ray powder diffraction pattern with few sharp peaks.

As used herein the term "pseudopolymorph" refers to a hydrate of a compound. In other words it is a crystal form that incorporates a stoichiometric amount of water.

As used herein, an effective amount of a compound for treating a particular disease is an amount that is sufficient to ameliorate, or in some manner reduce the symptoms associated with the disease.

As used herein, the term 'thermodynamically stable' refers to a crystal form that, during storage under long-term conditions (25°C, 60% relative humidity), substantially does not convert into another one for a pharmaceutically acceptable period of time (at least 3 months, preferably 6 months, more preferably 1 year).

As used herein, the term 'high level of chemical purity' refers to a crystal form wherein the total amount of readily detectable impurities as determined by standard methods of analysis, such as thin layer chromatography (TLC) or high performance liquid chromatography (HPLC), used by those of skill in the art to assess such purity, is less than 5%, advantageously less than 2.5%, preferably less than 1.0, more preferably less than 0.5% w/w.

As used herein, the term "high level of crystallinity" refers to a crystal form wherein the percentage of crystallinity is equal to or higher than 90%, preferably higher than 95% w/w as determined by standard methods of analysis used by those of skill in the art, such as X-ray powder diffraction or microcalorimetry.

### BRIEF DESCRIPTION OF THE DRAWINGS

Figure 1 is the differential scanning calorimetry (DSC) thermal trace of crystal form E.
Figure 2 is the X-ray powder diffraction (XRPD) pattern of crystal form E.
Figure 3 is the Raman spectrum of crystal form E.
Figure 4 shows the comparison among diffraction patterns of crystal form E at different temperatures.
Figure 5 is the comparative DSC thermal trace of the crystalline form A.
Figure 6 is the comparative XRPD pattern of the crystal form A.

### DETAILED DESCRIPTION OF THE INVENTION

The present invention provides a thermodynamically stable crystalline form of CHF 4226 having a high level of chemical purity and cristallinity, designated crystal form E.

Said form is a pseudopolymorph. TGA results indeed are compatible with a hemihydrate form (weight loss at 100°C of 2.3% vs theoretic of 2.17%).

Crystal form E can be characterised in a variety of ways.

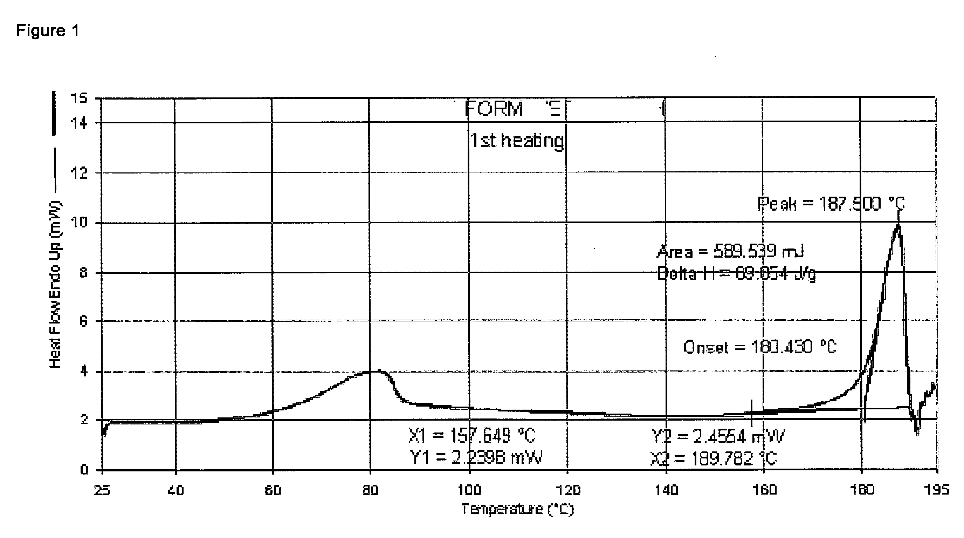

Its thermal trace, shown in Figure 1, exhibits an endothermic peak starting from about 60°C corresponding to the loss of water, and a melting peak at 187.5°C.

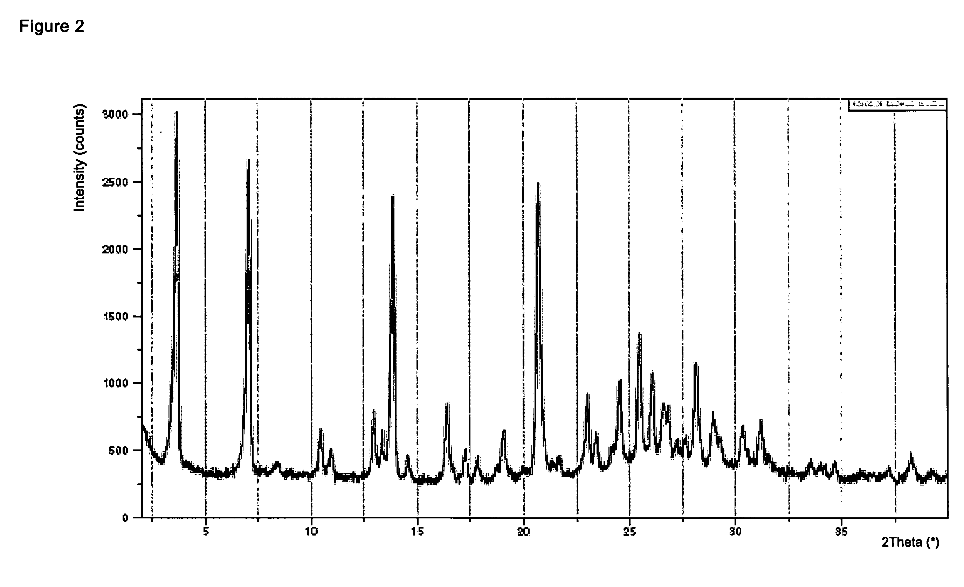

Crystal form E has the characteristic diffraction lines in the XPRD pattern thereof shown in Figure 2.

Said characteristic diffraction lines are reported in Table 1.

**Table 1**

| Pos. [°2Th.] | Height [cts] | FWHM [°2Th.] | d-spacing [Å] | Rel. Int. [%] |
|---|---|---|---|---|
| 6,6788 | 2581,23 | 0,1338 | 24,01840 | 100,00 |
| 7,0724 | 2293,50 | 0,1338 | 12,49917 | 88,85 |
| 8,4119 | 65,11 | 0,2007 | 10,51160 | 2,52 |
| 10,4693 | 316,53 | 0,1673 | 8,45004 | 12,26 |
| 10,9811 | 183,72 | 0,1338 | 8,05731 | 7,12 |
| 12,9646 | 457,87 | 0,1338 | 6,82874 | 17,74 |
| 13,3680 | 325,70 | 0,0669 | 6,62353 | 12,62 |
| 13,8888 | 1932,03 | 0,1171 | 6,37631 | 74,85 |
| 14,5762 | 165,44 | 0,1004 | 6,07712 | 6,41 |
| 16,4415 | 564,14 | 0,1840 | 5,39163 | 21,86 |
| 17,2926 | 203,52 | 0,1338 | 5,12814 | 7,88 |
| 17,8561 | 162,79 | 0,1338 | 4,96758 | 6,31 |
| 19,0818 | 342,47 | 0,1673 | 4,65115 | 13,27 |
| 20,7188 | 2031,20 | 0,1338 | 4,28722 | 78,69 |
| 21,6713 | 106,70 | 0,1338 | 4,10089 | 4,13 |
| 23,0375 | 556,30 | 0,1506 | 3,86069 | 21,55 |
| 23,4358 | 233,06 | 0,1673 | 3,79597 | 9,03 |
| 24,5585 | 545,83 | 0,1338 | 3,62493 | 21,15 |
| 25,4934 | 913,99 | 0,1673 | 3,49407 | 35,41 |
| 26,0876 | 599,44 | 0,1673 | 3,41582 | 23,22 |
| 26,6159 | 371,17 | 0,1673 | 3,34921 | 14,38 |
| 26,8630 | 335,62 | 0,1004 | 3,31896 | 13,00 |
| 27,6583 | 123,46 | 0,1673 | 3,22531 | 4,78 |
| 28,1362 | 698,94 | 0,0669 | 3,17161 | 27,08 |
| 28,9456 | 329,83 | 0,2007 | 3,08473 | 12,78 |
| 30,3573 | 281,04 | 0,1673 | 2,94443 | 10,89 |
| 31,2203 | 324,81 | 0,2342 | 2,86498 | 12,58 |
| 33,5418 | 80,51 | 0,2007 | 2,67181 | 3,12 |
| 34,6835 | 114,72 | 0,2007 | 2,58643 | 4,44 |

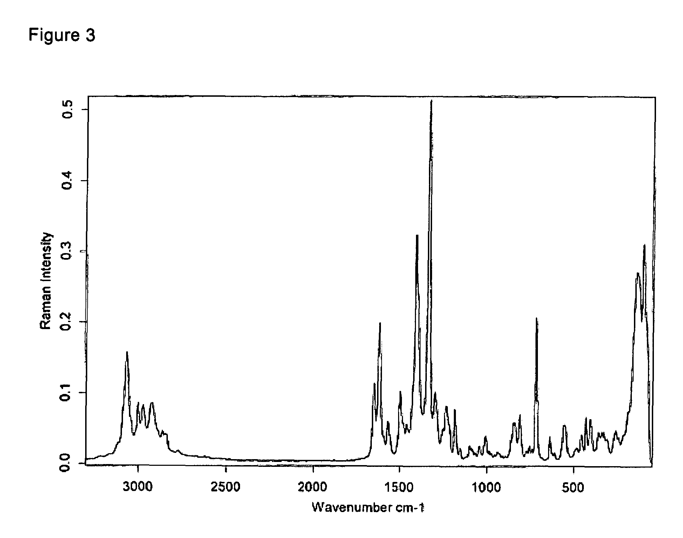

Crystal form E may also be characterised by its FT-Raman spectrum. The FT-Raman spectrum of crystal form E is shown in Figure 3. The mean peaks in the Raman spectrum are reported as follows: 3068 cm⁻¹ (m), 1648 (w), 1618 (m), 1408 (s), 1397 (sh), 1335 (vs), 718 (m).

Crystal form E is a hemihydrate.

This may be important in view of the sensitivity of CHF 4226 to moisture when formulating medicaments that comprise it in the solid form.

Without being limited by theory, it may indeed be hypothesized that crystal form E, having water incorporated in its unit crystal cell, may tend to absorb less moisture from the environment.

The present invention also provides a process for the preparation of said crystal form, which process comprises crystallising raw CHF 4226 from a solution thereof in a solvent or a mixture of solvents under conditions which yield crystal form E.

The precise conditions under which said form is obtained may be empirically determined and it is only possible to give a number of methods which have been found to be suitable in practice.

In general the crystal form of the invention may be prepared by crystallisation under particular conditions of raw CHF 4226 obtained as reported in EP 147719 or by re-crystallisation of the previously isolated crystal form A or by re-crystallization of any other crystal forms which may become known in the future.

Thus, for example, crystal form E may be prepared by crystallization of raw CHF 4226 from a solution thereof in a solvent kept at the refluxing temperature, said solvent comprising a mixture of a non aqueous solvent miscible with water and water in a 1:1 ratio v/v.

Advantageously the non aqueous solvent is selected from the group consisting of methanol, acetone: and acetonitrile.

Otherwise, isolated CHF 4226 crystal form A is suspended at room temperature in one of the aforementioned solvent mixtures and the resulting slurry is stirred until form E crystals are formed.

Advantageously the slurry is stirred for a period varying from 12 hours to 6 or 7 days.

The crystal form of the invention is readily isolable and may be filtered off from the crystallisation medium, optionally after washing and drying.

If desired, the obtained crystal form may further be re-crystallised using crystallisation conditions similar to those previously described.

The crystal form of the invention may be formulated for administration in any convenient way and the invention refers also to pharmaceutical compositions comprising CHF 4226 crystal form E.

The skilled person can establish without undue experimentation the effective dosage and/or the concentration of the active ingredient in the composition for a specific therapeutic purpose.

In the compositions provided herein, an effective concentration of the crystal form E is mixed with one or more suitable pharmaceutical carriers vehicles or excipients, for example those described in Remington's Pharmaceutical Sciences Handbook, Mack. Pub., N.Y., USA.

The concentration of the crystal form E in the formulation is effective for delivery, upon administration, an amount sufficient to exert a therapeutically useful effect.

The compositions may also contain, if required, one or more other therapeutic agents, preferably those currently used in the treatment of respiratory disorders, e.g. corticosteroids such as budesonide and its epimers, beclometasone dipropionate, triamcinolone acetonide, fluticasone propionate, flunisolide, mometasone furoate, rofleponide and ciclesonide, anticholinergic or antimuscarinic agents such as ipratropium bromide, oxytropium bromide, tiotropium bromide, glycopyrrolate bromide, and the group of phosphodiesterase-4 (PDE-4) inhibitors such as roflumilast.

The crystal form of the invention may be formulated for oral, buccal, topical, parenteral, vaginal, rectal or inhalation administration.

Inhalation administration is particularly preferred.

Upon said route of administration, the dose regimen is twice or once daily, where the suitable dose is advantageously in the range of 0.5 to 8 µg, preferably of 1 to 4 µg, more preferably of 2 to 4 µg.

Inhalable preparations include inhalable powders, propellant-containing metering aerosols or propellant-free inhalable solution or suspension formulations.

Advantageously, the inhalable powder formulations for inhalation comprise the crystal form of the invention under the form of interactive ordered mixtures.

More advantageously said formulations comprise a fraction of coarse particles of a physiologically acceptable excipient such as alpha-lactose monohydrate, said particles having a mass median diameter (MMD) higher than 90 micron, preferably the mass diameter (MD) comprised between 50 micron and 500 micron, more preferably between 150 and 400 micron, even more preferably between 210 and 355 micron.

Preferably said powder formulations further comprise a fraction of microparticles obtained by co-milling having a MMD lower than 35 micron, and constituted of particles of a physiologically acceptable excipient and an additive material selected from the class of the anti-adherents such as the amino acids leucine and isoleucine or of the lubricants such as magnesium stearate; sodium stearyl fumarate stearyl alcohol, stearic acid and sucrose monopalmitate.

More preferably said powder formulations comprise a fraction of microparticles having a MMD lower than 15 micron, preferably lower than 10 micron, constituted of particles of alpha-lactose monohydrate and particles of magnesium stearate.

Even more preferably, the inhalable powder formulations comprising the crystal form of the invention are prepared according to the teaching of co-pending application no. PCT/IB2007/0038924.

In general, CHF 4226 crystal form E may be used in preparation of a medicament for any disease or condition in which it is found therapeutically effective.

Having regard to its beta₂-adrenoceptor stimulating activity, CHF 4226 crystal form E is useful in the relaxation of bronchial smooth muscle and the relief of bronchoconstriction. Relief of bronchoconstriction can be measured in models such as the in vivo guinea pigs model (see Kikkawa et al Biol Pharm Bull 1994, 17(8), 1047-1052) and analogous models.

Administration of the crystal form of the invention may be indicated for the prevention and/or treatment of mild, moderate or severe acute or chronic symptoms or for prophylactic treatment of respiratory diseases such as asthma and chronic obstructive pulmonary disease (COPD). Other respiratory disorders characterized by obstruction of the peripheral airways as a result of inflammation and presence of mucus such as chronic obstructive bronchiolitis and chronic bronchitis may also benefit from their use.

The invention is further illustrated by the following examples.

Examples 1 and 2 deal with the preparation of crystal form E, examples 3 and 4 deal with stability experiments, example 5 refers to an exemplary formulation, and example 6 refers to the comparative characteristics of crystal form A.

### EXAMPLES

### Example 1 - Preparation of CHF 4226 crystal form E by crystallization

Crystallization is performed in a 500 ml jacketed glass reactor connected to thermocryostat, so that temperature could be accurately controlled; besides, crystallization is followed using a Lasentec FBRM D600 probe, so that nucleation and crystals growth could be monitored.

30 g of raw CHF4226 obtained as reported in EP 147719 is suspended in 250 ml of acetonitrile at refluxing temperature, and then 250 ml of water (50:50 v/v) is added portionwise until dissolution is complete.

The obtained solution is left at room temperature overnight.

After 20 hours, a solid sample is collected, filtered dried at T=40°C under vacuum, and analyzed for solid state, i.e. by XRPD, Raman, DSC and TGA.

### Methods of analysis

### 1. X-ray powder diffraction (XRPD)

The XRPD analyses are carried out on a PANanalytical X'pert Pro X-ray powder diffractometer using Cu Ka radiation. The instrument is equipped with a X'Celerator detector.

A theta-two theta continuous scan from 2.5 degrees 2 theta to 40 degrees 2 theta is used. Each sample is prepared for analysis by placing it in a quartz sample holder.

The XPRD patterns are reported in terms of degrees 2 theta (°2φ, accuracy ± 0.1°), diffraction peak intensity (height, [cts]), full width at half maximum (FWHM, [°2Th]), interatomic spacing in angstroms (d-spacing, [Ǻ]), and relative intensities (%). The relative intensity is recorded as the ratio of the peak intensity to that of the most intense peak.

### 2. Raman spectra

The Raman spectra are acquired on a FT-Raman Bruker IFS66 spectrophotometer equipped with a FRA-106 FT-Raman module and a cooled Ge-diode detector.

The spectra are scanned in the range 3350-50 cm⁻¹.

The main peaks in wavenumbers (accuracy ± 1 cm⁻¹) and the relative intensities* are reported.
*Legend: vs = very strong, s = strong; m = medium; w = weak; sh = shoulder

### 3. Differential scanning calorimetry (DSC)

The differential scanning calorimetry data are obtained on a Diamon Perkin Elmer Instrument.

The calibration standard used is indium. Approximately 2 to 5 mg of a sample is placed into a DSC pan and the weight is accurately measured and recorded. The pan is hermetically sealed. The sample is heated under nitrogen at a rate of 10°C/min, from 25°C to a final temperature of 200°C.

### 4. Thermogravimetric analysis (TGA)

The thermogravimetric analysis (TGA) is carried out on a Pyris 7 Perkin Elmer instrument.

Approximately 2 to 5 mg of a sample is placed in the pan, accurately weighed and inserted into the TG furnace. The sample is then heated in nitrogen at a rate of 10°C/min, from 25°C to decomposition of the sample.

The results of the DSC, XRPD and Raman analysis are shown in Figures 1, 2 and 3.

TGA is compatible with a hemihydrate form (weight loss at 100°C of 2.3% vs theoretic of 2.17%).

### Example 2 - Re-crystallization of CHF 4226 crystal form A to give CHF 4226 crystal form E

CHF 4226 crystal form A is obtained according to the Example of co-pending application WO 2005/089760.

5 g of sample is suspended in 200 ml of water: methanol 50:50 v/v for 1 week at room temperature.

The suspension is filtered.

The collected solid sample is dried and analyzed for solid state (XRPD, TGA, Raman and DSC) as described in Example 1.
Purity (HPLC): > 99%.

### Example 3 - Stability studies

In order to check its thermodynamic stability, the CHF 4226 crystal form E is stored at room temperature under different relative humidity (RH) conditions:
- 40% RH. and 25°C for 1 week
- 60% RH. and 25°C for 1 week
- 80% RH and 25°C for 1 week
- 60% RH and 25°C for 1 week
- 40% RH and 25°C for 1 week
- 30% RH and 25°C for 1 week
- 40% RH and 25°C for 1 week.

The thermodynamic stability of all the samples is checked by recording their XPRD pattern.

Crystal form E shows stable features under all stability screening conditions.

### Example 4 - Variable temperature stability studies

The variable temperature experiments are carried out on CHF 4226 crystal form E as said pseudopolymorph could theoretically produce phase transition due to the loss of water.

XPRD patterns are collected every 5-6 minutes, changing the temperature of the sample holder contained in a climatic device of the diffractometer from 25°C to 100°C.

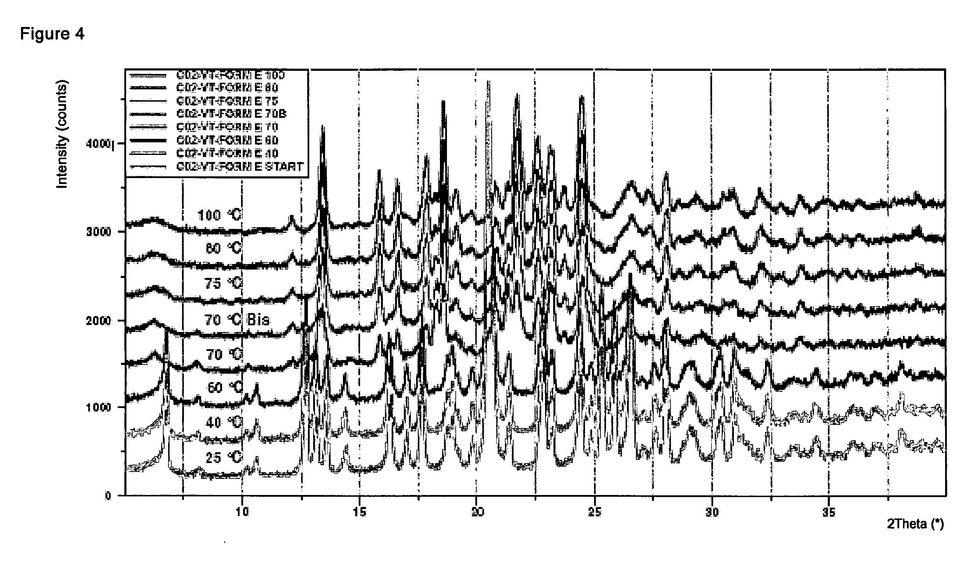

The comparison between the XPRD patterns at different temperatures is reported in Figure 4.

A phase transition is observed to another form when the sample is heated at about 60-70°C.

The phase transition is related to the loss of water content from crystal form E.

### Example 5 - Exemplary inhalable dry powder formulation comprising CHF 4226 crystal form E.

The composition is reported in Table 2.

**Table 2**

| Components | Amounts | | |
|---|---|---|---|
| | Per shot of the inhaler | | Single dose |
| | mg | % | µg |
| CHF 4226 crystal form E | 0.004 | 0.04 | 4 |
| alpha-lactose monohydrate 212-355 µm | 8.996 | 89.96 | |
| microparticles of alpha-lactose monohydrate and magnesium stearate obtained by co-milling | 1.00 | 10.0 | |
| Total weight | 10 | | |

### Comparative Example 6

CHF 4226 crystal form A is prepared according to the Example of pending application WO 2005/089760.

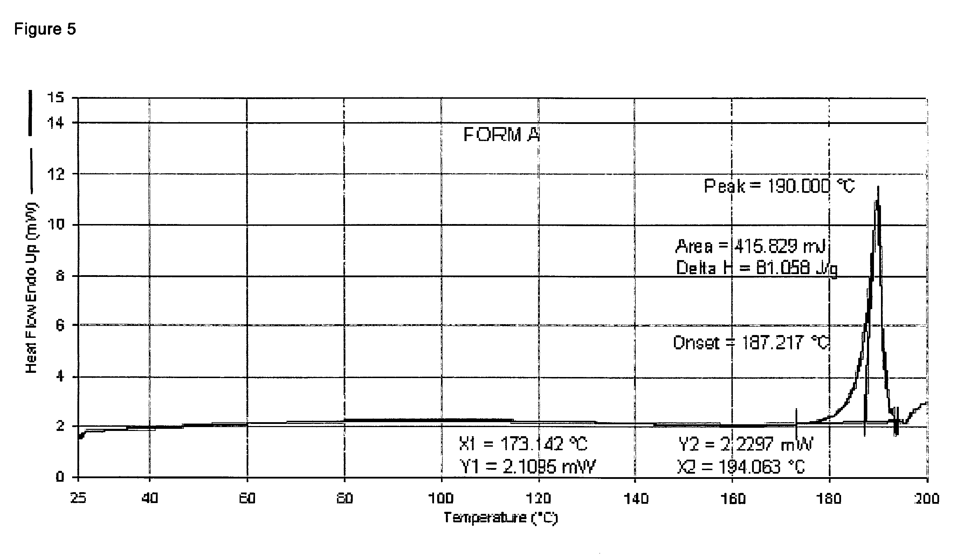

Its thermal trace, shown in Figure 5, exhibits a melting peak at 190.0°C.

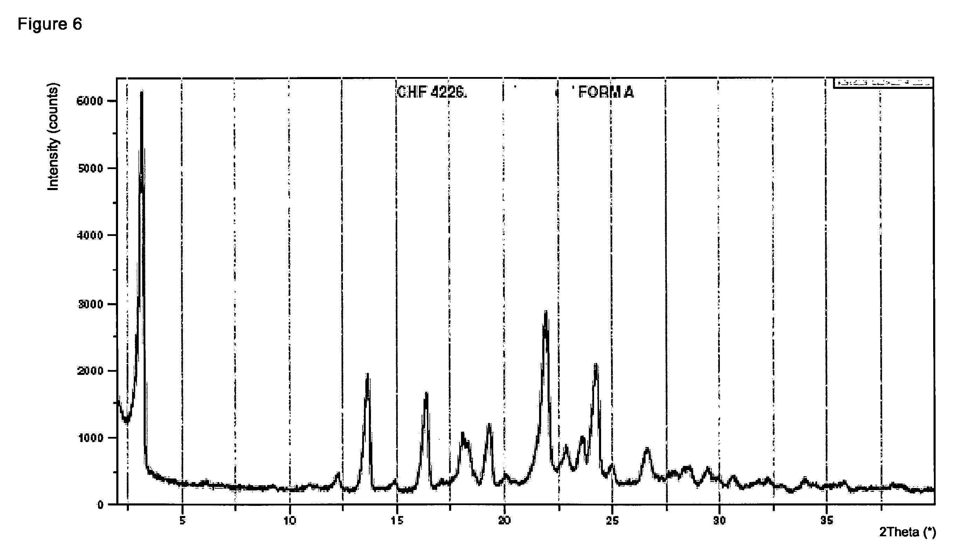

Crystal form A has the characteristic diffraction lines in the XPRD pattern thereof shown in Figure 6.

Said characteristic diffraction lines are reported in Table 3.

**Table 3**

| Pos. [°2Th.] | Height [cts] | FWHM [°2Th.] | d-spacing [Ǻ] | Rel. Int. [%] |
|---|---|---|---|---|
| 3,2171 | 4882,25 | 0,1673 | 27,46407 | 100,00 |
| 6,1648 | 56,30 | 0,2007 | 14,33719 | 1,15 |
| 11,0017 | 48,41 | 0,5353 | 8,04228 | 0,99 |
| 12,2932 | 221,92 | 0,2007 | 7,20012 | 4,55 |
| 13,6849 | 1628,41 | 0,2007 | 6,47093 | 33,35 |
| 14,9235 | 119,56 | 0,2007 | 5,93650 | 2,45 |
| 16,4033 | 1404,05 | 0,2007 | 5,40410 | 28,76 |
| 17,0392 | 72,21 | 0,2676 | 5,20383 | 1,48 |
| 18,0629 | 735,52 | 0,1673 | 4,91115 | 15,07 |
| 18,3442 | 579,41 | 0,1673 | 4,83647 | 11,87 |
| 19,4085 | 652,26 | 0,0836 | 4,57360 | 13,36 |
| 20,0887 | 115,52 | 0,2007 | 4,42025 | 2,37 |
| 22,0423 | 1972,56 | 0,2175 | 4,03270 | 40,40 |
| 22,9299 | 403,55 | 0,1171 | 3.87856 | 8,27 |
| 23,6648 | 552,77 | 0,2007 | 3,75976 | 11,32 |
| 24,3181 | 1607,95 | 0,2175 | 3,66022 | 32,93 |
| 25,0090 | 237,34 | 0,1673 | 3,56064 | 4,86 |
| 28,6681 | 452,07 | 0,3346 | 3,34302 | 9,26 |
| 28,6123 | 224,79 | 0,4015 | 3,11990 | 4,60 |
| 29,532 | 204,45 | 0,2342 | 3,02478 | 4,19 |
| 30,6165 | 147,68 | 0,2676 | 2,92007 | 3,92 |
| 32,2879 | 129,66 | 0,2007 | 2,77264 | 2,66 |
| 33,9670 | 131,82 | 0,2676 | 2,63932 | 2,70 |
| 35,8516 | 103,81 | 0,2676 | 2,50479 | 2,13 |
| 38,2891 | 50,71 | 0,6891 | 2,35076 | 1,04 |

The results indicate that crystal form E is clearly distinguishable from crystal form A of CHF 4226.

## Claims

1. Crystalline 8-hydroxy-5-[(1R)-1-hydroxy-2-[[(1R)-2-(4-methoxyphenyl)-1-methylethyl]amino]ethyl]-2(1H)-quinolinone monohydrochloride (CHF 4226) hemihydrate, designated crystal form E.

2. The crystalline 8-hydroxy-5-[(1R)-1-hydroxy-2-[[(1R)-2-(4-methoxyphenyl)-1-methylethyl]amino]ethyl]-2(1H)-quinolinone monohydrochloride as claimed in claim 1 having the following characteristic XRPD peaks at 2φ (accuracy ± 0.1°): 3.68, 7.07, 8.41, 10.47, 10.98, 12.96, 13.37, 13.89, 14.58, 16.44, 17.29, 17.86, 19.08, 20.72, 21.67, 23.04, 23.44, 24.56, 25.49, 26.09, 26.62, 26.86, 27.66, 28.14, 28.95, 30.36, 31.22, 33.54, and 34.68.

3. The crystalline 8-hydroxy-5-[(1R)-1-hydroxy-2-[[(1R)-2-(4-methoxyphenyl)-1-methylethyl]amino]ethyl]-2(1H)-quinolinone monohydrochloride as claimed in claim 1 or claim 2 characterised inter alia by peaks in the Raman spectrum at 3068 cm⁻¹, 1648, 1618, 1408, 1397, 1335, 718.

4. A process for preparing crystal form E of any one of claims 1 to 3 which comprises the step of crystallising raw 8-hydroxy-5-[(1R)-1-hydroxy-2-[[(1R)-2-(4-methoxyphenyl)-1-methylethyl]amino]ethyl]-2(1H)-quinolinone monohydrochloride from a solution thereof in a solvent kept under the refluxing temperature under conditions which yield crystal form E.

5. The process as claimed in claim 4 wherein the solvent comprises a mixture of a non aqueous solvent miscible with water and water in a 1:1 ratio v/v.

6. The process as claimed in claim 5 wherein the non aqueous solvent is selected from the group consisting of methanol, acetone and acetonitrile.

7. A pharmaceutical composition comprising the crystal form E of claims 1 to 3, in admixture with a pharmaceutically acceptable carrier.

8. The pharmaceutical composition as claimed in claim 7 further comprising a therapeutic agent selected from the group consisting of corticosteroids, anticholinergic or antimuscarinic agents and phosphodiesterase-4 (PDE-4) inhibitors.

9. The pharmaceutical composition as claimed in claim 7 or 8 wherein the pharmaceutical composition is an inhalable aerosol comprising a propellant.

10. The pharmaceutical composition as claimed in claim 7 or 8 wherein the pharmaceutical composition is an inhalable powder.

11. Use of the crystal form E of any one of claims 1 to 3, as a medicament.

12. Use of the crystal form E of any one of claims 1 to 3 for the prevention and/or treatment of an inflammatory or obstructive airways disease.

13. Use of the crystal form E of any one of claims 1 to 3, in the manufacture of a medicament for the prevention and/or treatment of an inflammatory or obstructive airways disease.
